(19) Europäisches Patentamt
European Patent Office
Office européen des brevets

(11) Publication number: **0 021 685**
**A1**

(12) # EUROPEAN PATENT APPLICATION

(21) Application number: 80301913.2

(22) Date of filing: 06.06.80

(51) Int. Cl.³: C 07 C 103/52
A 61 K 37/02

(30) Priority: 08.06.79 US 46875
01.02.80 US 117739

(43) Date of publication of application: 07.01.81 Bulletin 81/1

(84) Designated Contracting States: DE GB LU NL SE

(71) Applicant: ELI LILLY AND COMPANY
307, East McCarty Street
Indianapolis Indiana(US)

(72) Inventor: Michel, Karl Heinz
225 East North Street, Apt 2205
Indianapolis Indiana(US)

(74) Representative: McVey, Kenneth William Henry et al,
Erl Wood Manor
Windlesham Surrey, GU20 6PH(GB)

(54) **A-30912 H-type antibiotics, their preparation and use.**

(57) A process for the preparation of antibiotic A-30912 factor H, its lower alkyl ether homologs and the tetrahydro derivatives of its homologs having the structural formula

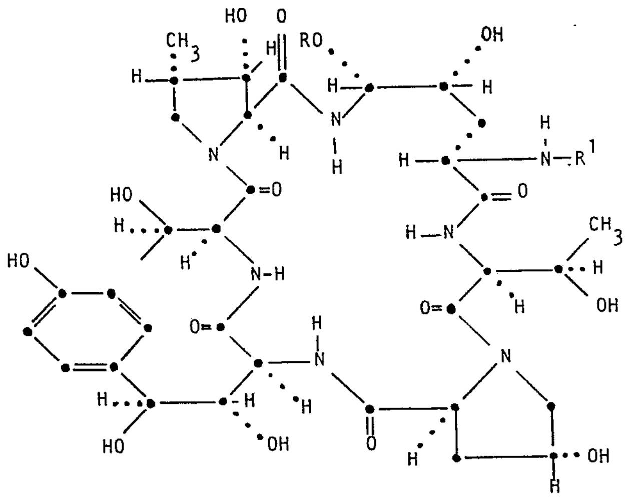

wherein $R^1$ is linoleoyl or stearoyl and R is $C_1$-$C_6$ alkyl, subject to the proviso that when R is methyl, $R^1$ is linoleoyl characterized by reacting antibiotic A-30912 factor A or its tetrahydro derivative with an acidic lower alkanol.

EP 0 021 685 A1

A-30912 H-TYPE ANTIBIOTICS, THEIR PREPARATION AND USE.

This invention relates to A-30912 factor H, a new antibiotic factor isolated from the A-30912 antibiotic mixture, to the lower alkyl ether homologs of A-30912 factor H, and to the tetrahydro derivatives of the homologs of this new factor. These novel compounds have the structural formula I:

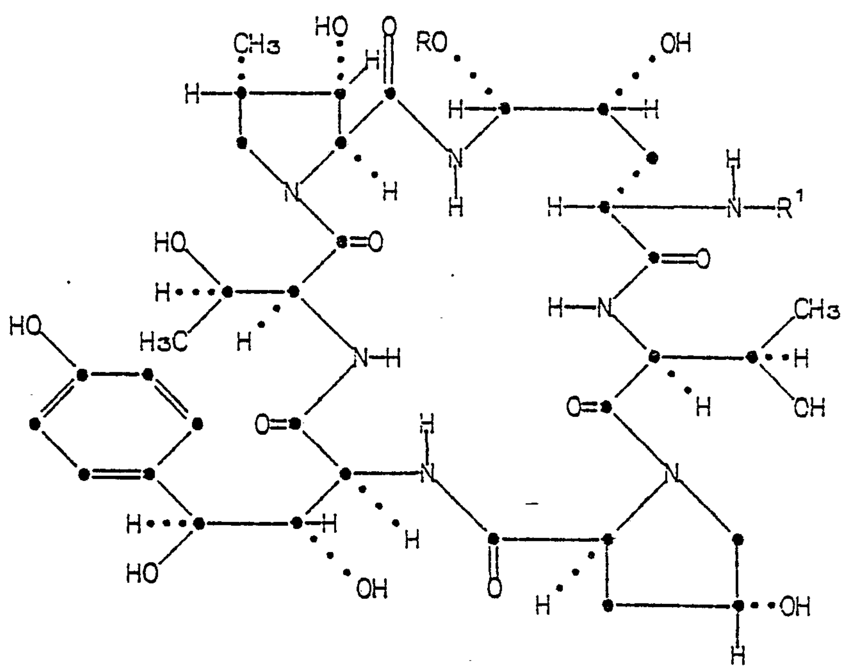

I

wherein $R^1$ is linoleoyl or stearoyl and R is $C_1$-$C_6$ alkyl, subject to the proviso that when R is methyl, $R^1$ is linoleoyl. When $R^1$ is linoleoyl and R is methyl, the compound is A-30912 factor H; when $R^2$ is stearoyl and R is methyl, the compound is tetrahydro-A-30912 factor H.

These compounds are prepared by reacting A-30912 factor A or tetrahydro A-30912 factor A with the appropriate alcohol. The tetrahydro derivatives can also be made by reducing the corresponding linoleoyl compound.

The A-30912 antibiotic mixture is described by Marvin M. Hoehn and Karl H. Michel in U.S. Patent 4,024,245. Prior to the present invention, seven individual factors of the A-30912 mixture had been recognized. These factors were designated A-30912 factors A, B, C, D, E, F, and G. The newly isolated antibiotic substance of this invention has been designated A-30912 factor H. For convenience herein, A-30912 factor H will be abbreviated A-30912H; and A-30912H, the lower alkyl homologs of A-30912H, and their tetrahydro derivatives will be called A-30912H-type antibiotics.

The purpose of this invention is the disclosure of A-30912H-type antibiotics of formula I and a process for preparing them from the A-30912 factor A or the tetrahydro A-30912 factor A. The A-30912H-type antibiotics are especially useful as antifungal agents.

This invention provides an A-30912H antibiotic which has the structural formula:

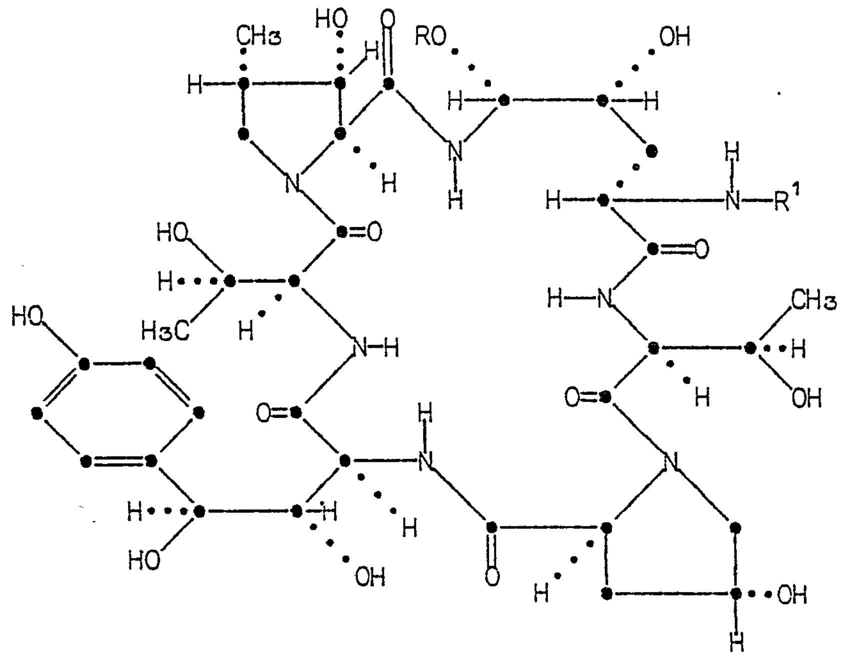

I

wherein $R^1$ is linoleoyl or stearoyl and R is $C_1$-$C_6$ alkyl, subject to the proviso that when R is methyl $R^1$ is linoleoyl.

This invention also provides a process for preparing an A-30912H-type antibiotic which has the structural formula I, as before described, wherein $R^1$ is linoleoyl or stearoyl and R is $C_1$-$C_6$ alkyl, subject to the proviso that when R is methyl $R^1$ is linoleoyl, characterized by reacting a compound of Formula II

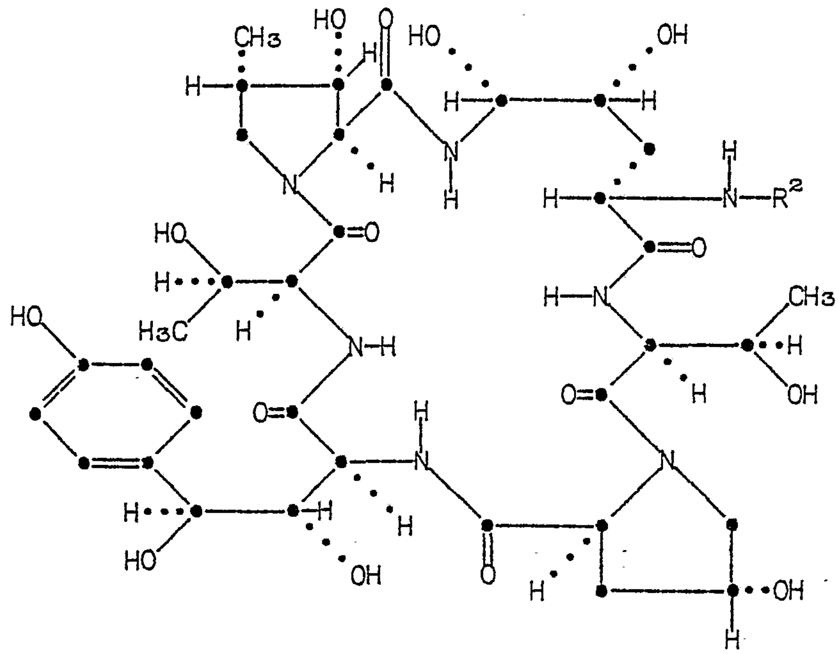

II

wherein $R^2$ is linoleoyl or stearoyl

A) when $R^2$ is linoleoyl, with

1) an alcohol of the formula ROH wherein R is defined as above; or

2) an alcohol of the formula $R^3OH$ wherein $R^3$ is $C_2$-$C_6$ alkyl;

B) when $R^2$ is stearoyl, with an alcohol of the formula $R^3OH$;

in a polar inert solvent or in an excess of the reactant alcohol at 0°C to 70°C. under acidic conditions; and when the alcohol $R^3OH$ is reacted with a compound of Formula II wherein $R^2$ is linoleoyl, optionally, reducing the linoleoyl group in the resulting compound to stearoyl.

This invention also provides a pharmaceutical formulation which comprises the active ingredient, an

A-30912H-type antibiotic which has the structural formula I as before described, associated with a pharmaceutically acceptable carrier therefor.

This invention also provides an A-30912H-type antibiotic which has the structural formula I as before described for use as an antifungal agent for treating fungal infections in mammals by the administration of an effective amount thereof.

The A-30912 antibiotic mixture, from which the new antibiotic factor of this invention can be isolated, is produced by culturing a strain of *Aspergillus rugulosus* NRRL 8113 under submerged aerobic fermentation conditions as described in U.S. Patent 4,024,245. Another method for preparing A-30912H is by culturing *Aspergillus nidulans* var. *roseus*, NRRL 11440, to give the A-42355 antibiotic mixture and isolating A-30912H from this mixture. This process is described in the copending application of LaVerne D. Boeck and Ralph E. Kastner entitled PROCESS OF PRODUCING THE A-30912 ANTIBIOTICS.

## Description of the Drawings

The infrared absorption spectrum of A-30912 factor H in KBr disc is presented in Figure 1 of the accompanying drawings.

Figure 2 of the accompanying drawings summarized the relative movements of the A-30912 factors on thin-layer chromatography (TLC), using silica gel (Merck-Darmstadt) adsorbent, an ethyl acetate:methanol (3:2) solvent system, and *Candida albicans* bioautography for detection. The arrow indicates the direction of

solvent flow; the "+" indicates the point of origin. The area where minor factors E, F, and G may be seen is indicated by bracketing. Specific $R_f$ comparisons between A-30912 factor A and minor factors E, F, and G are found in U.S. Patent 4,024,245 (see Table I in column 4).

## Detailed Description

This invention relates to new antifungal agents. These agents include A-30912H, a newly discovered factor from the A-30912 antibiotic mixture, lower alkyl homologs of A-30912H and the tetrahydro derivatives of these homologs. This invention further relates to the method of using tetrahydro-A-30912H as an antifungal agent. The tetrahydro derivatives of A-30912H and its homologs are prepared by reduction of these compounds.

The A-30912 antibiotic mixture can be prepared as described in U.S. Patent 4,024,245, which is incorporated herein by reference. The A-30912 antibiotic mixture can also be prepared by culturing *Aspergillus nidulans* var. *roseus*, NRRL 11440, as herein described.

A-30912H is a new factor of the A-30912 antibiotic mixture. The term "antibiotic mixture" as used in the fermentation art and in this specification refers to a mixture of co-produced individual antibiotic factors. As will be recognized by those familiar with antibiotic production by fermentation, the ratio of individual factors produced in an antibiotic mixture will vary, depending on the fermentation conditions

used. Under conditions known thus far, A-30912H is a minor factor in the A-30912 mixture, being present in amounts in the range of from 0.01 to 1.0 percent of the total mixture. Another minor factor of the A-30912 mixture has been recognized, but has not been isolated in an amount sufficient for characterization. A-30912 factor H is best separated from this factor by silica-gel TLC using an ethyl acetate:methanol (3:2) or an acetonitrile:water (95:5) solvent system. In either system, the minor factor is more polar than the other A-30912 factors.

A. A-30912 Factor H

A-30912 factor H, the newly discovered component of the A-30912 antibiotic mixture, is a polypeptide antibiotic which is quite similar to A-30912 factor A. Recently, evidence has shown that A-30912 factor A is identical to echinocandin B [see F. Benz *et al.*, *Helv. Chim. Acta 57*, 2459-2477 (1974) and Swiss Patent 568,386 (Derwent Abstract 75884W)]. Antibiotic SL 7810/F has also been identified as echinocandin B [see C. Keller-Juslen, *et al.*, *Tetrahedron Letters 1976* (46), 4147-4150, and Belgium Patent 834,289 (Derwent Abstract 30159X)].

Keller-Juslen, *et al.*, proposed structures 2 and 3 for echinocandin B (SL 7810/F; A-30912 factor A) and its tetrahydro-derivative, respectively:

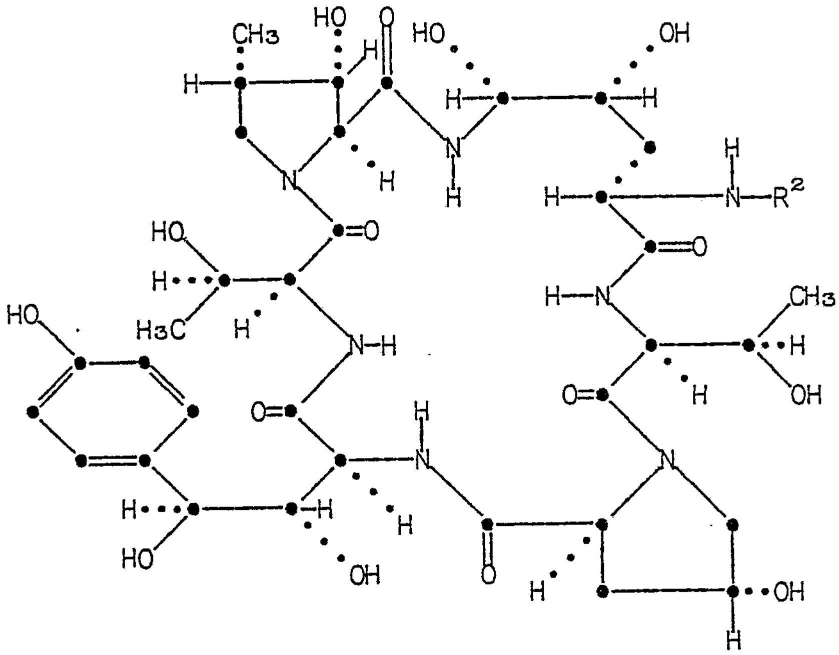

II

2 $R^2$ = linoleoyl: echinocandin B
3 $R^2$ = stearoyl: tetrahydro-echinocandin B

The elemental analysis of A-30912 factor H corresponds especially well with the empirical formula of $C_{53}H_{83}N_7O_{16}\cdot H_2O$ (Calcd.: C, 58.30; H, 7.79; N, 8.98; O, 24.93).

The infrared absorption spectrum of A-30912 factor H in KBr disc is shown in Figure 1 of the accompanying drawings. The following characteristic absorption maxima are observed: 2.9 (very strong), 3.4 (strong), 3.5 (medium), 5.9-6.1 (very strong), 6.6 (strong), 6.9 (strong), 7.9-8.1 (medium), and 9.1 (strong) microns.

The ultraviolet absorption spectra of A-30912 factor H in both neutral and acidic methanol exhibit absorption maxima at 223 nm (ε 13,100) and 275 nm, broad peak (ε 2,100). The ultraviolet spectrum of

A-30912 factor H in basic methanol shows absorption maxima at 245 nm (ε 14,700) and 290 nm, broad peak (ε 3,500), and also end absorption.

The $^{13}C$ nuclear magnetic resonance spectrum of A-30912 factor H in perdeuteromethanol shows the following characteristics:

δ 176.07, 174.15, 173.49, 172.56, 172.47, 169.88, 158.45, 133.01, 130.90, 129.52, 129.04, 116.21, 82.15, 77.00, 75.98, 75.71, 71.27, 69.55, 69.42, 68.22, 62.44, 58.69, 57.25, 56.81, 56.08, 52.88, 51.32, 39.08, 38.60, 36.89, 32.65, 30.77, 30.45, 30.26, 28.18, 27.14, 26.55, 20.11, 19.60, 11.33.

A-30912 factor H has the following approximate specific rotations:

$[\alpha]_D^{25}$ -40° ($\underline{c}$ 0.5, $CH_3OH$)

$[\alpha]_{365}^{25}$ -151° ($\underline{c}$ 0.5, $CH_3OH$)

Electrometric titration of A-30912 factor H in 66% aqueous dimethylformamide indicated the presence of a titratable group with a $pK_a$ value of about 12.90 (initial pH 7.12).

A-30912 factor H is soluble in a variety of organic solvents such as methanol, ethanol, dimethylformamide, dimethyl sulfoxide, and ethyl acetate; but is insoluble in nonpolar organic solvents such as diethyl ether and petroleum ether. A-30912 factor H is also soluble in aqueous solutions, especially those having a pH greater than 7.0.

A-30912 factor H (mol. wt. 1073) differs from A-30912 factor A (mol. wt. 1059) by only 14 mass units. Both compounds are very similar in physical-chemical properties, and both compounds produce linoleic acid upon hydrolysis. A-30912H has an additional $-CH_2-$ which is present as $-O-CH_3$, replacing one of the -OH groups in the cyclic peptide portion of the molecule.

A-30912H and tetrahydro-A-30912H have structures 4 and 5, respectively:

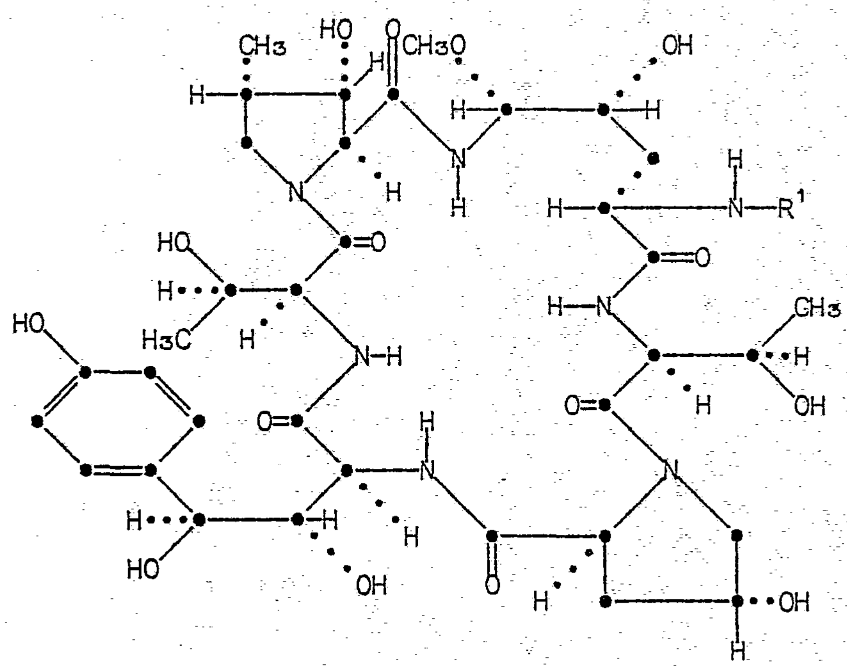

III

4 $R^1$ = linoleoyl
3 $R^1$ = stearoyl

B. Homologs of A-30912 Factor H

Following the discovery of the structure of A-30912 factor H, the fact that lower alkyl ($C_2$-$C_6$) ether homologs of A-30912 factor H would be useful products became appreciated. Prior to this time, the lower alkyl ether derivatives of A-30912 factor A which had been prepared were not recognized as having a useful purpose and were prepared only as structure determination tools. The lower alkyl ether homologs of A-30912 factor H are prepared from A-30912 factor A.

A-30912 factor A may be produced by fermentation of: 1) a strain of *Aspergillus rugulosus* NRRL 8113 as described in U.S. Patent 4,024,245; 2) a strain of *Aspergillus nidulans* NRRL 8112 as described in U.S. Patent 4,024,246; 3) a strain of *Aspergillus nidulans* var. *echinulatus* A-32204, NRRL 3860 as described in Swiss Patent 568,386; or 4) a strain of *Aspergillus rugulosus* NRRL 8039 as described in Belgian Patent 834,289.

Another method for preparing A-30912 factor A by fermentation of *Aspergillus nidulans* var. *roseus* NRRL 11440 is described in the co-pending Boeck et al. application.

C. Preparation of A-30912 Factor H

A-30912 factor H is isolated from the A-30912 antibiotic mixture. The A-30912 mixture can be prepared as described in U.S. Patent 4,024,245.

Alternatively, A-30912 factor H can be isolated from the A-42355 mixture. A-42355 mixture is

prepared by culturing a new variety of *Aspergillus nidulans*, named *A. nidulans* var. *roseus*, as herein described. This culture has been deposited and made a part of the stock culture collection of the Northern Regional Research Center U.S. Department of Agriculture, Agricultural Research, Peoria, Illinois 61604, from which it is available to the public under the number NRRL 11440. A sample of the same microorganism has been deposited in the culture collection of the American Type Culture Collection on 5th June 1980 under the number ATCC 20600.

## Taxonomy of *Aspergillus nidulans* var. *roseus*

The *Aspergillus nidulans* var. *roseus* culture which is useful for producing the A-30912 antibiotics was studied and characterized by Thomas H. Sands of the Lilly Research Laboratories.

For convenience herein, this new culture will be called culture A42355. The taxonomic basis upon which this culture is classified as a new variety of *Aspergillus nidulans* is discussed in the following paragraphs. In this discussion, the terms "ISCC-NBS" refer to color names based on the ISCC-NBS method (K. L. Kelly and D. B. Judd, "The ISCC-NBS Methods of Designating Colors and A Dictionary of Color Names," U.S. Department of Commerce, Circ. 553, Washington, D.C., 1955). The term "Maerz and Paul" refers to color blocks described by A. Maerz and M. R. Paul in "Dictionary of Color," McGraw-Hill Book Company, New York, N.Y., 1950.

Comparisons are based on the work of K. B. Raper and D. I. Fennel, "The Genus *Aspergillus*," Williams and Wilkins, 1965.

Culture A42355 attains a diameter of 14 mm in seven days and 35 mm in 21 days when grown on Czapek's solution agar at 25°C. The colony surface is radially buckled, convex and initially velutinous to slightly floccose. The surface appearance changes with age due to the formation of a pink exudate that, after drying occurs, results in a pocked surface. The margin is strongly crenate to lobate and sharply delineated, showing only scant peripheral subsurface growth. As the colony ages, pigment variation causes a zonate effect. The reverse surface appears concave and in contact with the agar only in the periphery. A pink soluble pigment is produced. The mycelial mat is very tough and produces no distinctive odor. Pigmentation in young colonies is influenced by immature conidia which are light greenish yellow (ISCC-NBS 101 and Maerz and Paul 11-K-1). This color eventually is confined to the colony periphery. After 10 days, subspherical masses of hülle cells encrusting blackish purple cleistothecia are scattered throughout the colony but are most noticable clustered in the center. In 21 days, the narrow, slightly flattened margin is grayish greenish yellow (ISCC-NBS 105 and Maerz and Paul 12-I-2). Inside this margin, the colony is moderate yellowish pink (ISCC-NBS 29 and Maerz and Paul 11-A-6), and the color is influenced by the exudate. After 21 days, the center is darkened by grayish green conidia and hülle-cell-encrusted blackish purple cleistothecia. The reverse ranges from light brown (ISCC-NBS 57 and Maerz and Paul 13-A-7) to moderate reddish brown

(ISCC-NBS 43 and Maerz and Paul 6-F-9). In three weeks or more, the reverse is in shades of very dark purple, nearly black.

The conidial head at first is radiate and bright yellow and then becomes dark green and tightly columnar. When mature, heads range from 93.4 μ to 116.7 μ long and have an average size of 106 μ x 47 μ. The vesicle is somewhat spathulate to pyriforme and, like the conidiophore, is light brown. Vesicles are fertile over the upper two-thirds and range in size from 10 to 12 μ x 8 to 10 μ (average size 9.2 μ x 11.3 μ). Conidiophores are sinuous, smooth and relatively thick-walled. They range in length from 88 μ to 112 μ (average 101 μ). Although they range in width from 4 to 6 μ, most are 6 μ wide.

Sterigmata are biseriate, hyaline to light brown and are smooth-walled. Primary sterigmata are nearly cuneiform. They are from 6.3 μ to 10.3 μ long and from 2 μ to 3 μ wide at their widest point (average size: 9.3 μ x 2.7 μ). Secondary sterigmata are between ovoid and obpyriform and range from 5.5 μ to 11.0 μ x from 2.4 μ to 3.2 μ (average size: 9.9 μ x 2.9 μ). Conidia are globose, delicately roughened to echinulate and dark green. They range from 2.4 to 4.0 μ and average 3.2 μ in diameter.

On malt-extract agar at 35°C, a 14-mm diameter colony is produced in seven days. The colony diameter will achieve 30 mm in 21 days. Initially, the colony consists of white aerial hyphae. Conidiation occurs in the first week, and the relatively flat, velutinous,

crenate colony becomes dark yellowish green (ISCC-NBS 137 and Maerz and Paul 24-J-6). The colony appears zonate due to concentric rings of yellow spherical masses of hülle cells that surround or encrust dark purple cleistothecia. The conidiogenous state on malt-extract agar resembles that state on Czapek's solution agar except for dimensions.

Conidial heads range in length from 100 μ to 170 μ and average 138 μ x 50 μ in size. Conidiophores are from 100 μ to 210 μ long (average 182 μ) and are 6 μ wide. Vesicles are from 8 μ to 12 μ x from 6 μ to 12 μ (average size: 12 μ x 9.2 μ). Primary sterigmata are from 5.5 μ to 8.7 μ x 2.4 μ to 4.0 μ (average size: 7.2 μ x 6.6 μ). Secondary sterigmata are 5.5 μ to 10.3 μ x 2.0 μ to 3.6 μ (average size: 7.4 μ x 3.5 μ). Conidia are from 2.3 to 3.2 μ, but average 3.2 μ, in diameter.

The ascogenous state is similar on both agars. Numerous dark purple cleistothecia are encrusted with globose to subglobose, thick-walled, hyaline-to-pale-yellow hülle cells which are from 12.6 μ to 18.2 μ in diameter (average size: 15.3 μ). Cleistothecia are globose to subglobose and have walls up to three layers thick, the outer layer consisting of pseudoparenchymatous cells. Cleistothecia range in diameter from 140 μ to 800 μ, but are mostly 165 μ to 250 μ with an average diameter of 229 μ. The eight-spored hyaline asci are globose to subglobose or irregularly ellipsoidal. When globose, they are 9.2 μ in diameter with little deviation from this size. Ellipsoidal asci are 9.3 μ x 8.3 μ. Ascospores are orange-red pigmented, globose in

the surface view and lenticular in the lateral view. Two delicately pleated, entire, parallel equatorial crests are seen in the long axis of the lenticular view. In the surface view, a single crest is seen peripheral to the main spore body which is smooth and of bivalve construction. The crest is 0.5 μ wide; the spore body is 4.4 μ in diameter. The average lenticular view size is 4.7 μ x 3.7 μ.

Yellowish green rugulose conidia, columnar conidial heads, smooth-walled light brown conidiophores and vesicles, biseriate sterigmata and the production of globose, thick-walled hülle cells place A42355 in the Aspergillus nidulans group. These characteristics, combined with the fact that the culture has an ascogenous state in which dark purple globose cleistothecia are closely associated with, or even encrusted with, hülle cells (as above) and has orange-red lenticular ascospores which are adorned with two pleated, parallel equatorial crests, place A42355 in the genus Emericella, the perfect state of the A. nidulans group.

A42355 does not fully fit the description of any of the published species or varieties in the A. nidulans group. The species most similar to A42355 is A. nidulans (Eidam) Wint., based on the type culture WB187 (NRRL 187). These cultures are similar in color and ascospore type, but differ in crest width. Cleistothecia are similar, but the wall of A42355 is a multilayer of cells instead of a single layer. Both cultures exhibit a yellow green, predominant conidial stage on malt-extract agar, but the ascogenous state of A42355 is more strongly evident than is that of the

type culture where it is partially overgrown and is dully pigmented. In both cultures, the hyphae, conidiophores and vesicles are smooth-walled, without encrustment; however, the vesicle of A42355 is spathulate to pyriforme, whereas the vesicle of A. nidulans is hemispherical. Colonies of A42355 and A. nidulans when grown on Czapek's solution agar differ in growth rate, exudate production, abundance and size of conidial heads and in most other measured dimensions.

Although there are similarities between A42355 and A. nidulans (Eidam) Wint., there are sufficient significant differences that A42355 is considered a new variety which has been designated A. nidulans var. roseus.

## Cultivation of A. rugulosus NRRL 8113

Cultivation conditions for A. rugulosus NRRL 8113 are described in U.S. Patent 4,024,245.

## Cultivation of A. nidulans var. roseus

The culture medium used to grow Aspergillus nidulans var. roseus can be any one of a number of media. For economy in production, optimal yield and ease of product isolation, however, certain culture media are preferred. Thus, for example, a preferred carbon source in large-scale fermentation is cottonseed oil or glucose, although molasses, starch, dextrin, lactose, sucrose, maltose, glycerol, fatty acids and the like may be used. Preferred nitrogen sources are enzyme-hydrolyzed casein, soybean meal and soluble meat

peptone, although distiller's grains, nitrate salts, monosodium glutamate and the like may be used. Nutrient inorganic salts can be incorporated in the culture media. These include the customary soluble salts capable of yielding sodium, magnesium, zinc, iron, calcium, ammonium, chloride, carbonate, sulfate, nitrate, phosphate and the like.

Essential trace elements necessary for the growth and development of the organism should also be included in the culture medium. Such trace elements commonly occur as impurities in other constituents of the medium in amounts sufficient to meet the growth requirements of the organism.

It may be necessary to add small amounts (i.e., 0.2 ml/L) of an antifoam agent such as polypropylene glycol to large-scale fermentation media if foaming becomes a problem.

For production of a substantial quantity of the A-30912 antibiotic mixture, submerged aerobic fermentation in tanks is preferred. Small quantities of the A-30912 antibiotic mixture may be obtained by shake-flask culture. Because of the time lag in antibiotic production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture of the organism. The vegetative inoculum is then transferred to a larger tank. The

medium used for the growth of the vegetative inoculum can be the same as that used for larger fermentations, but other media can also be used.

*A. nidulans* var. *roseus* can be grown at temperatures between 20° and 43°C; the organism grows best at temperatures of 30-37°C. Optimum production of the A-30912 antibiotic mixture occurs at temperatures 30°C.

As is customary in aerobic submerged culture processes, sterile air is blown through the culture medium. For efficient antibiotic production, there should be sufficient aeration and agitation to maintain a dissolved oxygen level of at least 40% of air saturation at atmospheric pressure. Agitation is also helpful in breaking up the thick and heavy culture growth during the fermentation.

Production of the A-30912 antibiotic mixture can be followed during the fermentation by testing samples of fermented broth or alcoholic extracts of the biomass or the whole broth for antibiotic activity against an organism known to be sensitive to the A-30912 antibiotics. One assay organism useful in testing for the presence of the A-30912 antibiotic mixture is *Candida albicans*. The bioassay is conveniently performed by paper-disc or agar-well assay on seeded agar plates.

Generally, antibiotic activity can be detected by the second day of fermentation. Maximum production of antibiotic activity usually occurs between about the sixth and the eighth days.

A-30912 factor H can be identified and separated from the remainder of the A-30912 mixture by

TLC. Silica gel is a preferred adsorbent. Figure 2 of the drawings shows the movement of A-30912H in relationship to that of the other A-30912 factors in a silica-gel TLC system. The approximate $R_f$ values of A-30912 factors A, B, C, D, and H in different solvent systems, using silica gel TLC (Merck-Darmstadt silica gel #60 plates, 20 x 20 cm) and detecting via Candida albicans bioautography, are given in Table I.

TABLE I

| A-30912 Factor | $R_f$ Values - Solvent Systems | | | |
|---|---|---|---|---|
| | a | b | c | d |
| Factor A | 0.28 | 0.14 | 0.28 | 0.43 |
| Factor B | 0.39 | 0.21 | 0.42 | 0.47 |
| Factor C | 0.46 | 0.31 | 0.51 | 0.58 |
| Factor D | 0.50 | 0.38 | 0.57 | 0.61 |
| Factor H | 0.42 | 0.27 | 0.36 | 0.53 |

Solvent Systems

a: ethyl acetate:methanol (3:2)

b: ethyl acetate:methanol (7:3)

c: acetonitrile:water (95:5)

d: ethyl acetate:ethanol:acetic acid (40:60:0.25)

A-30912 factor H can be recovered from the fermentation medium by methods known in the fermentation art. Maximum recovery is accomplished by extraction of the whole broth. It is preferable to add an equal volume of methanol to the whole broth and to lower the pH of the whole broth to about pH 4-6 before extraction. Chloroform is an especially advantageous solvent for extracting A-30912 factor H, although other such solvents may be used.

A-30912 factor H may be isolated from the separated A-30912 or A-42355 mixture by chromatography using various adsorbents. Suitable adsorbents include silica gels such as Quantum LP-1; reversed-phase resins such as silica gel/$C_8$, silica gel/$C_{18}$, LiChroprep RP-8 or RP-18; Florisil; Sephadex G-25, LH-20, and G-15; alumina; Diaion HP-20; and Amberlite XAD-4 and X-384. Diaion is available from Mitsubishi Chemical Industries, Tokyo; the Amberlite resins are available from Rohm and Haas Co., Philadelphia, Pa.; the Sephadex resins are available from Pharmacia Fine Chemicals AB, Uppsala, Sweden; Florisil is available from Floridin Co., Tallahassee, Fla; and silica gel/$C_8$, silica gel $C_{18}$, LiChroprep RP-8 and RP-18 are available from E. Merck, Darmstadt, Germany. The preparation of a high loading capacity silica gel/$C_{18}$ from LP-1 silica gel (Whatman) is described in Example 6.

Reversed-phase high performance, low pressure liquid chromatography (HPLPLC) using silica gel/$C_{18}$ adsorbent is a preferred method for final purification of A-30912 factor H. In this method, the A-30912 or

A-42355 mixture (obtained, for example, by extraction of the whole broth or mycelia with methanol and chloroform), dissolved in methanol:water:acetonitrile (7:2:1), is placed on a column equilibrated with the same solvent. The column is then eluted with the same solvent. Fractions collected are monitored by *Candida albicans* bioautography and/or by UV (based on relative retention times). Fractions containing A-30912 factor H are combined. It is sometimes necessary to carry out an additional chromatographic separation in order to obtain A-30912 factor H in purified form.

A-30912 factors A, B, D and H can be separated by HPLPLC using the following conditions:

| | |
|---|---|
| Column: | glass, 0.8 x 15.0 cm |
| Packing: | Nucleosil® 10-$C_{18}$ (Machery-Nagel and Company); packed using slurry-packing procedure of Example 7 |
| Solvent: | methanol:water:acetonitrile (7:2:1) |
| Sample Volume: | 8 mcl |
| Sample Size: | 8 mcg |
| Column Temperature: | ambient |
| Flow Rate: | 1.8 ml/min |
| Pressure: | ca. 200 psi |
| Detector: | UV at 222 nm (ISCO Model 1800 Variable Wavelength UV-Visible Absorbance Monitor) |
| Pump: | LDC Duplex Minipump |
| Injection: | loop injection |

The approximate retention times for A-30912 factors A,

B, D, and H under these conditions are summarized in Table II.

Table II

| A-30912 Factor | Retention Time (seconds) |
|---|---|
| A | 792 |
| B | 870 |
| H | 990 |
| D | 1,140 |

Tetrahydro-A-30912H can be prepared by standard hydrogenation techniques, carrying out the reduction until both double bonds of the linoleoyl side chain have been reduced.

D. Preparation of the A-30912H Homologs

The A-30912H antibiotics of formula I wherein R is $C_2$-$C_6$ alkyl (the A-30912H homologs) are prepared by reaction of A-30912 factor A or tetrahydro-A-30912A with the appropriate corresponding alcohol to prepare the desired $C_2$-$C_6$ ether derivative. Since A-30912 factor A is the major component of the antibiotic mixtures in which it is produced, this method is also a preferred way of preparing A-30912 factor H and tetrahydro-A-30912H.

The A-30912H homologs of formula I wherein $R^1$ is stearoyl and R is $C_2$-$C_6$ alkyl can be prepared by a) preparing tetrahydro-A-30912A and reacting with the appropriate alcohol to form the ether derivative, or b) reacting A-30912 factor A with the appropriate alcohol to form the ether derivative and then reducing the double bonds of the linoleoyl side chain.

Tetrahydro-A-30912A, tetrahydro-A-30912H, and the compounds of formula I wherein R is $C_2$-$C_6$ and $R^1$ is stearoyl are prepared from A-30912 factors A and H and from the compounds of formula I wherein R is $C_2$-$C_6$ and $R^1$ is linoleoyl by standard hydrogenation techniques, carrying out the reduction until both double bonds of the linoleoyl side chain have been reduced.

The A-30912H-type antibiotics are antifungal agents which are especially active against Candida species. This invention relates further, therefore, to a method of treating fungal infections in mammals which comprises administering to the mammal an effective amount of an A-30912H-type antibiotic. The results of tests which illustrate this antifungal activity are summarized in Tables III and IV. In these tests the antifungal activity was measured by the disc-diffusion method (6-mm pads were dipped in solutions containing test compounds; pads were placed on agar plates seeded with the test organism). Table III gives the minimal inhibitory concentration (MIC) per disc at which A-30912H inhibited the test organism.

TABLE III

| Test Organism | MIC (mcg/disc) |
|---|---|
| Candida albicans | 1.25 |
| Trichophyton mentagrophytes | 0.078 |

Table IV gives the diameters (in mm) of the observed zones of inhibition for typical A-30912H-type derivatives. The test compounds were compounds of formula I wherein $R^1$ is linoleoyl; the pads were (dipped in solutions containing 10 mg/ml of test compound).

TABLE IV

| Test Organism | Zone diameters (mm) | |
|---|---|---|
| | R = n-propyl | R = n-hexyl |
| *Neurospora crassa* | 20 | 25 |
| *Candida albicans* | 16 | 14 |
| *Trichophyton mentagrophytes* | 35 | 35 |

In addition, in standard agar-dilution tests A-30912H showed MIC's of 100 mcg/ml against both *Blastomyces dermatitidis* and *Histoplasma capsulatum*.

The A-30912H-type antibiotics are relatively non-toxic. When used as antifungal agents, A-30912H-type antibiotics are administered parenterally and are commonly administered together with a pharmaceutically acceptable carrier or diluent. The dosage used will depend upon a variety of conditions such as the nature and severity of the particular infection involved.

In order to illustrate this invention more fully, the following examples are provided.

## EXAMPLE 1

### Preparation of A-30912 Factor H from A-42355 Mixture

#### A. Shake-Flask Fermentation

A culture of *Aspergillus nidulans* var. *roseus* NRRL 11440 is prepared and maintained on an 18- x 150-ml agar slant prepared with medium having the following composition:

| Ingredient | Amount |
| --- | --- |
| Glucose | 5 g |
| Yeast extract | 2 g |
| $CaCO_3$ | 3 g |
| Vegetable juice* | 200 ml |
| Agar** | 20 g |
| Deionized water | q.s. to 1 liter |

(initial pH 6.1)

*V-8 Juice, Campbell Soup Co., Camden, N.J.

**Meer Corp.

The slant is inoculated with Aspergillus nidulans var. roseus NRRL 11440, and the inoculated slant is incubated at 25°C. for about seven days. The mature slant culture is covered with water and scraped with a sterile loop to loosen the spores. The resulting suspension is further suspended in 10 ml of sterile deionized water. One ml of the suspended slant growth is used to inoculate 55 ml of vegetative medium in a 250-ml flask. The vegetative medium has the following composition:

| Ingredient | Amount |
| --- | --- |
| Sucrose | 25 g |
| Blackstrap molasses | 36 g |
| Corn-steep liquor | 6 g |
| Malt extract | 10 g |
| $K_2HPO_4$ | 2 g |
| Enzymatic hydrolysate of casein* | 10 g |
| Tap water | 1100 ml |

(initial pH 6.5-6.7)

*N-Z-Case, Humko Sheffield Chemical, Lyndhurst, N.J.

The inoculated vegetative medium is incubated at 25°C. for 48 hours at 250 rpm on a rotary-type shaker. After 24 hours, the medium is homogenized for one minute at low speed in a blender (Waring type) and then returned to incubation for the remaining 24 hours. Alternatively, the inoculated vegetative medium can be incubated for 48 hours and then homogenized for 15 seconds at low speed.

This incubated vegetative medium may be used to inoculate shake-flask fermentation culture medium or to inoculate a second-stage vegetative medium. Alternatively, it can be stored for later use by maintaining the culture in the vapor phase of liquid nitrogen. The culture is prepared for such storage in multiple small vials as follows:

The vegetative cultures are mixed volume/volume with a suspending solution having the following composition:

| Ingredient | Amount |
|---|---|
| Glycerol | 20 ml |
| Lactose | 10 g |
| Deionized water | q.s. to 100 ml |

The prepared suspensions are distributed in small sterile screw-cap tubes (4 ml per tube). These tubes are stored in the vapor phase of liquid nitrogen.

A stored suspension thus prepared can be used to inoculate either agar slants or liquid seed media. Slants are incubated at 25°C. in the light for 7 days.

B. Tank Fermentation

In order to provide a larger volume of inoculum, 10 ml of incubated first-stage vegetative culture is used to inoculate 400 ml of a second-stage vegetative growth medium having the same composition as that of the vegetative medium. The second-stage medium is incubated in a two-liter wide-mouth Erlenmeyer flask at 25°C. for 24 hours on a shaker rotating through an arc two inches in diameter at 250 rpm.

Incubated second-stage medium (800 ml), prepared as above described, is used to inoculate 100 liters of sterile production medium selected from one of the following:

MEDIUM I

| Ingredient | Amount |
|---|---|
| $ZnSO_4 \cdot 7H_2O$ | 0.00455 g/L |
| Soluble meat peptone* | 30.5 g/L |
| Soybean meal | 15.5 g/L |
| Tapioca dextrin** | 2.0 g/L |
| Blackstrap molasses | 10.5 g/L |
| Enzymatic hydrolysate of casein*** | 8.5 g/L |
| $Na_2HPO_4$ | 4.5 g/L |
| $MgSO_4 \cdot 7H_2O$ | 5.5 g/L |
| $FeSO_4 \cdot 7H_2O$ | 0.1 g/L |
| Cottonseed oil | 40.0 ml |
| (Antifoam)**** | 1.0 ml |
| Tap water | 1000.0 ml |

(initial pH 6.8-7.0)

*O.M. Peptone, Amber Laboratories, Juneau, Wisc.

**Stadex 11, A.E. Staley Co., Decatur, Ill.

***N-Z-Amine A, Humko Sheffield Chemical, Lyndhurst, N.J.

****P2000, Dow Corning, Midland, Mich.

MEDIUM II

| Ingredient | Amount |
|---|---|
| Glucose | 2.5% |
| Starch | 1.0% |
| Soluble meat peptone* | 1.0% |
| Blackstrap molasses | 1.0% |
| $CaCO_3$ | 0.2% |
| $MgSO_4 \cdot 7H_2O$ | 0.05% |
| Enzymatic hydrolysate of casein** | 0.4% |
| (Antifoam)*** | 0.02% |
| Tap water | q.s. to volume |

*O.M. Peptone

**N-Z-Amine A

***Antifoam "A", Dow Corning

The inoculated production medium is allowed to ferment in a 165-liter fermentation tank at a temperature of 25°C. for about 7 days. The fermentation medium is aerated with sterile air, maintaining the dissolved oxygen level above 50 percent of air saturation.

C. Third-Stage Vegetative Medium

Whenever the fermentation is carried out in tanks larger than those used for 100-liter fermentation, it is recommended that a third-stage vegetative culture be used to seed the larger tank. A preferred third-stage vegetative medium has the following composition:

| Ingredient | Amount |
|---|---|
| Sucrose | 25 g |
| Blackstrap molasses | 25 g |
| Corn-steep liquor | 6 g |
| Enzymatic hydrolysate of casein* | 10 g |
| Malt extract | 10 g |
| $K_2HPO_4$ | 2 g |
| Tap water | 1000 ml |

(initial pH 6.1)

*N-Z-Case

D. Separation of the A-42355 Mixture

Whole fermentation broth (4127 liters), obtained by the method described in example 1 using production medium II, was stirred thoroughly with methanol (4280 liters) for one hour and then was filtered, using a filter aid (Hyflo Super-cel, a diatomaceous earth, Johns-Manville Products Corp.). The pH of the filtrate was adjusted to pH 4.0 by the addition of 5 N HCl. The acidified filtrate was extracted twice with equal volumes of chloroform. The chloroform extracts were combined and concentrated under vacuum to a volume of about 20 liters. This concentrate was added to about 200 liters of diethyl ether to precipitate the A-42355 mixture. The precipitate was separated by filtration to give 2775 g of the A-42355 mixture as a gray-white powder.

## EXAMPLE 2

Isolation of A-30912 Factor H from A-42355 Mixture

A-42355 antibiotic mixture (5.0 g), prepared as described in Example 1, was dissolved in 35 ml of methanol:water:acetonitrile (7:2:1); the resulting solution was filtered and introduced onto a 3.7-cm I.D. x 42-cm glass column (Michel-Miller Column) through a loop with the aid of a valve system. The column was packed with LP-1/$C_{18}$ silica gel reversed phase resin (10-20 microns) in methanol:water:acetonitrile (7:2:1) as described in Example 7. The solvent was moved through the column at a flow rate of 13 ml/min at ca. 120 psi as described in Example 3, collecting one fraction every two minutes. Elution of the antibiotic was monitored at 280 nm as described in Example 3. Fractions 112-132 were combined with fractions 106-117 from a second similar purification. The combined fractions were concentrated under vacuum to an oil. The oil was dissolved in a small volume of tert-butanol and lyophilized to give 173 mg of crude A-30912 factor H.

The crude A-30912 factor H (150 mg) was dissolved in 8 ml of methanol:water:acetonitrile (7:2:1); the resulting solution was filtered and introduced onto a 2.0-cm I.D. x 32-cm glass column, as described above. The solvent was moved through the column at a flow rate of 8 ml/min at ca. 80 psi as described in Example 3, collecting one fraction every three minutes. Elution of the antibiotic was monitored at 280 nm as described in Example 3. Fractions 17 and 18 were combined and

concentrated under vacuum to give an oil. The oil was dissolved in a small volume of *tert*-butanol and lyophilized to give 29 mg of A-30912 factor H.

EXAMPLE 3

Isolation of A-30912 Factor H from A-30912 Mixture

A-30912 antibiotic mixture (6 g), prepared as described in U.S. Patent 4,024,245, was dissolved in 33 ml of methanol:water:acetonitrile (7:2:1), filtered and introduced onto a 4.7-cm I.D. x 45-cm glass column [Michel-Miller High Performance Low Pressure (HPLPLC) Chromatography Column, Ace Glass, Inc., Vineland, NJ 08360] through a loop system with the aid of a valve system. The column was packed with LP-1/$C_{18}$ silica gel reversed-phase resin (10-20 microns), described in Example 6. Packing was accomplished in methanol:water:acetonitrile (7:2:1) by the slurry-packing procedure described in Example 7. An F.M.I. pump with valveless piston design (maximum flow 19.5 ml/min) was used to move the solvent through the column at a flow rate of 14 ml/min (100 psi), collecting fractions having a volume of approximately 14 ml. Elution of the antibiotic was monitored at 280 nm, using a UV monitor (ISCO Model UA-5, Instrument Specialist Co., 4700 Superior Ave., Lincoln, Nebraska 68504) with an optical unit (ISCO Type 6).

Fractions 109-151 were combined and concentrated under vacuum to give an oil. The resulting oil was dissolved in *tert*-butanol (50 ml). This solution was lyophilized to give 2.927 g of A-30912 factor A.

Fractions 152-185 were combined and concentrated under vacuum to give an oil. The resulting oil was dissolved in a small volume of tert-butanol. This solution was lyophilized to give 46.6 mg of crude A-30912 factor H.

EXAMPLE 4

Purification of A-30912 Factor H

Partially purified A-30912 factor H (597 mg), prepared as described in Example 3, was dissolved in 3 ml of methanol:water:acetonitrile (7:2:1), filtered, and introduced onto a 3.7-cm I.D. x 35-cm glass column (Michel-Miller Column) through a loop with the aid of a valve system. The column was packed with LP-1/$C_{18}$ silica gel reversed-phase resin as described in Example 3 in methanol:water:acetonitrile (7:2:1). The solvent was moved through the column at a flow rate of 7.5 ml/min (100 psi), collecting fractions every minute. Elution of the antibiotic was monitored at 280 nm, using a UV monitor (ISCO Model UA-5) with an optical unit (ISCO Type 6).

Fractions 159-190 were combined and added to 100 ml of water. The pH of the solution was adjusted to 4.0 with 1N HCl. This solution was extracted twice with equal volumes of chloroform. The two chloroform extracts were combined and concentrated under vacuum to give an oily residue. This residue was dissolved in a solution of tert-butanol (20 ml) containing a few ml of methanol. The resulting solution was lyophilized to give 222 mg of purified A-30912 factor H.

EXAMPLE 5

Preparation of Tetrahydro-A-30912H

A-30912H, prepared as described in Example 2 or 4, is dissolved in ethanol. $PtO_2$ in absolute ethanol is reduced to form Pt, which in turn is used to reduce the A-30912H catalytically, using hydrogenation under positive pressure until the reaction is complete (about 2-3 hours). The reaction mixture is filtered and concentrated under vacuum. The residue is dissolved in a small amount of tert-butanol and lyophilized to give tetrahydro-A-30912H.

EXAMPLE 6

Preparation of Silica Gel/$C_{18}$ Reversed Phase Resin

Step 1: Hydrolysis

LP-1 silica gel (1000 g from Quantum Corp., now Whatman) is added to a mixture of concentrated sulfuric acid (1650 ml) and concentrated nitric acid (1650 ml) in a 5-L round-bottom flask and shaken for proper suspension. The mixture is heated on a steam bath overnight (16 hours) with a water-jacketed condenser attached to the flask.

The mixture is cooled in an ice bath and carefully filtered using a sintered-glass funnel. The silica gel is washed with deionized water until the pH is neutral. The silica gel is then washed with acetone (4 L) and dried under vacuum at 100°C. for 2 days.

Step 2: First Silylation

The dry silica gel from Step 1 is transferred to a round-bottom flask and suspended in toluene (3.5 L). The flask is heated on a steam bath for 2 hours to azetrope off some residual water. Octadecyltrichlorosilane (321 ml, Aldrich Chemical Company) is added, and the reaction mixture is refluxed overnight (16 hours) with slow mechanical stirring at about 60°C. Care is taken so that the stirrer does not reach near the bottom of the flask. This is to prevent grinding the silica gel particles.

The mixture is allowed to cool. The silanized silica gel is collected, washed with toluene (3 L) and acetone (3 L), and then air-dried overnight (16-20 hours). The dried silica gel is suspended in 3.5 L of acetonitrile:water (1:1) in a 5 L flask, stirred carefully at room temperature for 2 hours, filtered, washed with acetone (3 L) and air-dried overnight.

Step 3: Second Silylation

The procedure from the first silylation is repeated using 200 ml of octadecyltrichlorosilane. The suspension is refluxed at 60°C. for 2 hours while stirring carefully. The final product is recovered by filtration, washed with toluene (3 L) and methanol (6 L), and then dried under vacuum at 50°C. overnight (16-20 hours).

EXAMPLE 7

Slurry Packing Procedure for Michel-Miller Columns

General Information

A. Analytical or preparative columns can be packed by this procedure.

B. Silica gels and silica gel reversed phase packings (e.g., Quantum LP-1, particle size 10-20 microns; LiChroprep RP-8 and RP-18, particle size 25-40 microns) are recommended. However, other silica gels (e.g., Shandons ODS Hypersil, particle size 5 microns) as well as other types of resins have been packed successfully by this procedure.

C. Generally, a pressure of less than 200 psi and flow rates between 5-40 ml/minute are required for this slurry packing technique; this is dependent on column volume and size. PLEASE NOTE: Packing pressure should exceed pressure used during actual separation by 30-50 psi; this will assure no further compression of the adsorbent during separation runs. Columns packed by this procedure with reversed-phase silica gel can be operated for several years without loss of efficiency.

D. Sudden decrease in pressure may cause cracks or channels to form in the packing material, which would greatly reduce column efficiency. Therefore, it is important to let the pressure drop slowly to zero whenever the pump has been turned off.

E. Approximate volume of columns (Ace Glass Cat. No., unpacked): 5795-04, 12 ml; 5795-10, 110 ml; 5795-16, 300 ml; 5795-24, 635 ml; and 5796-34, 34 ml.

F. The time required to pack a glass column will vary from minutes to several hours depending on column size and experience of the scientist.

Example:

1. Connect glass column to a reservoir column via coupling (volume of reservoir column should be twice that of the column). Place both columns in vertical positions (reservoir column above).

2. Weigh out packing material (100 g for 200 ml column).

3. Add five volumes of solvent to packing material; use a mixture of 70-80% methanol and 20-30% water.

4. Shake well until all particles are wetted, let stand overnight or longer to assure complete soaking of particles by solvent. Decant supernatant.

5. Slurry the resin with sufficient solvent to fill reservoir column. Pour swiftly into reservoir. NOTE: The column must be pre-filled with the same solvent and the reservoir column should be partly filled with solvent before slurry is poured. The use of larger slurry volumes may also provide good results; however, this will require (a) larger reservoir or (b) multiple reservoir fillings during the packing procedure.

6. Close reservoir with the Teflon plug beneath the column (see Figure 1 of U.S. Patent 4,131,547, plug No. 3); connect to pump; and immediately start pumping

solvent through system at maximum flow rate if Ace Cat. No. 13265-25 Pump or similar solvent-delivery system is used (20 ml/minute).

7. Continue until column is completely filled with adsorbent. Pressure should not exceed maximum tolerance of column during this operation (200 psi for large columns and 300 psi for analytical columns). In most cases, pressures less than 200 psi will be sufficient.

8. Should pressure exceed maximum values, reduce flowrate; pressure will drop.

9. After column has been filled with adsorbent, turn off pump; let pressure drop to zero; disconnect reservoir; replace reservoir with a pre-column; fill pre-column with solvent and small amount of adsorbent; and pump at maximum pressure until column is completely packed. For additional information, see general procedure.

NOTE: Always allow pressure to decrease slowly after turning off pump--this will prevent formation of any cracks or channels in the packing material.

10. Relieve pressure and disconnect pre-column carefully. With small spatula remove a few mm (2-4) of packing from top of column; place 1 or 2 filter(s) in top of column; gently depress to top of packing material, and place Teflon plug on top of column until seal is confirmed. Connect column to pump, put pressure on (usually less than 200 psi) and observe through glass wall on top of column if resin is packing any further. If packing material should continue to settle

(this may be the case with larger columns), some dead space or channelling will appear and step 9 should be repeated.

EXAMPLE 8

Preparation of A-30912 Factor H from A-30912 Factor A

A-30912 factor H is prepared from A-30912 factor A using the following procedure:

Antibiotic A-30912 factor A (19.6 mg) is dissolved in dimethylformamide (1 ml). Acidic methanol (3% HCl, 0.06 ml) is added to this solution. The resulting solution is stirred at room temperature overnight and then is evaporated to dryness under vacuum. The residue obtained is chromatographed by HPLPLC as described in Example 2, using reversed-phase silica gel (LP-1/$C_{18}$, prepared as described in Example 6) and $CH_3OH:H_2O:CH_3CN$ (7:2:1) as the eluting solvent to give 1.4 mg of A-30912 factor H (the compound of formula I wherein R is methyl and $R^1$ is linoleoyl).

EXAMPLE 9

The A-30912H-type antibiotic of structure I wherein R is ethyl and $R^1$ is linoleoyl is prepared and purified by the method of Example 8, except that ethanol is used in the reaction instead of methanol.

EXAMPLE 10

The A-30912H-type antibiotic having structure I wherein R is *n*-propyl and $R^1$ is linoleoyl is prepared and purified by the method of Example 8, except that *n*-propanol is used in the reaction instead of methanol.

EXAMPLE 11

The A-30912H-type antibiotic having structure I wherein R is isobutyl and $R^1$ is linoleoyl is prepared and purified by the method of Example 8, except that isobutanol is used in the reaction instead of methanol.

EXAMPLE 12

The A-30912H-type antibiotic of structure I wherein R is *n*-pentyl and $R^1$ is linoleoyl is prepared and purified by the method of Example 8, except that *n*-pentanol is used in the reaction instead of methanol.

EXAMPLE 13

The A-30912H-type antibiotic of structure I wherein R is *n*-hexyl and $R^1$ is linoleoyl is prepared and purified by the method of Example 8, except that *n*-hexanol is used in the reaction instead of methanol.

EXAMPLE 14

The A-30912H-type antibiotic having structure I wherein R is ethyl and $R^1$ is stearoyl is prepared and purified by the method of Example 8, except that tetrahydro-A-30912 factor A is used instead of A-30912 factor A and ethanol is used instead of methanol in the reaction.

EXAMPLE 15

The A-30912H-type antibiotic having structure I wherein R is 2-ethyl-1-butyl and $R^1$ is linoleoyl is prepared and purified by the method of Example 8, except that 2-ethyl-1-butanol is used instead of methanol in the reaction.

BAD ORIGINAL

EXAMPLE 16

The A-30912H-type antibiotic having structure I wherein R is 3-methyl-1-butyl and $R^1$ is linoleoyl is prepared and purified by the method of Example 8, except that 3-methyl-1-butanol is used instead of methanol in the reaction.

EXAMPLE 17

The A-30912-type antibiotic having structure I wherein R is n-propyl and $R^1$ is stearoyl is prepared and purified by the method of Example 10 except that tetrahydro-A-30912 factor A is used instead of A-30912 factor A.

EXAMPLE 18

The A-30912-type antibiotic having structure I wherein R is isobutyl and $R^1$ is stearoyl is prepared by hydrogenation of the compound wherein R is isobutyl and $R^1$ is linoleoyl (prepared as in Example 11) according to the method of Example 5.

CLAIMS

1. An A-30912H-type antibiotic which has the structural formula:

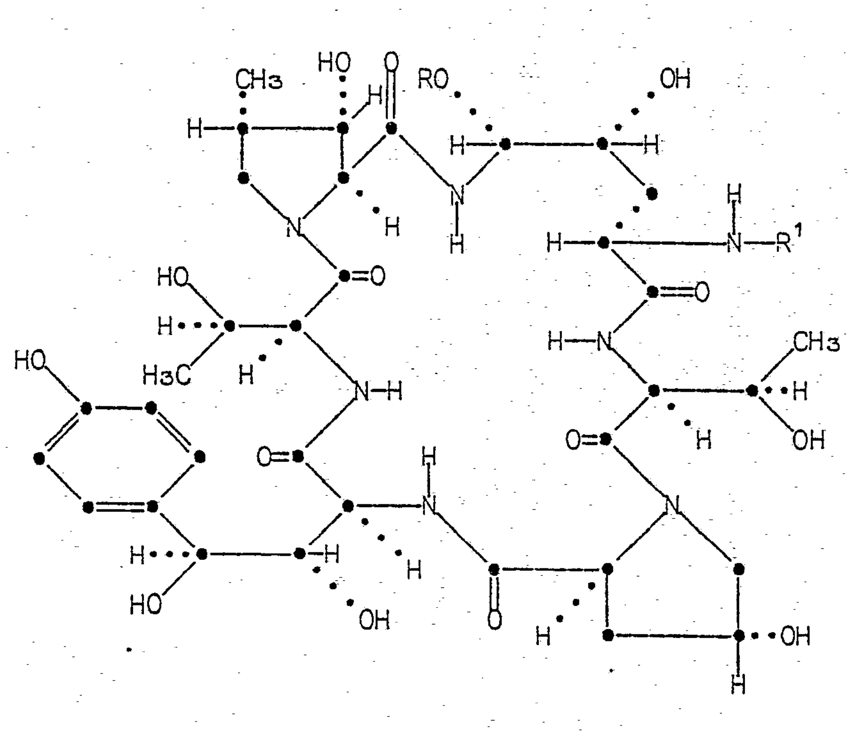

I

wherein $R^1$ is linoleoyl or stearoyl and R is $C_1$-$C_6$ alkyl, subject to the proviso that when R is methyl $R^1$ is linoleoyl.

2. The A-30912-type antibiotic of claim 1 wherein $R^1$ is linoleoyl and R is ($C_1$-$C_6$) alkyl.

3. The A-30912H-type antibiotic of claim 1 wherein $R^1$ is linoleoyl and R is methyl, that is, antibiotic A-30912 factor H.

4. The A-30912H-type antibiotic of claim 1 wherein $R^1$ is linoleoyl and R is propyl.

5. The A-30912H-type antibiotic of claim 1 wherein $R^1$ is linoleoyl and R is hexyl.

6. The A-30912H-type antibiotic of claim 1 wherein $R^1$ is stearoyl and R is $(C_2-C_6)$ alkyl.

7. A process for preparing an A-30912H-type antibiotic which has the structural formula:

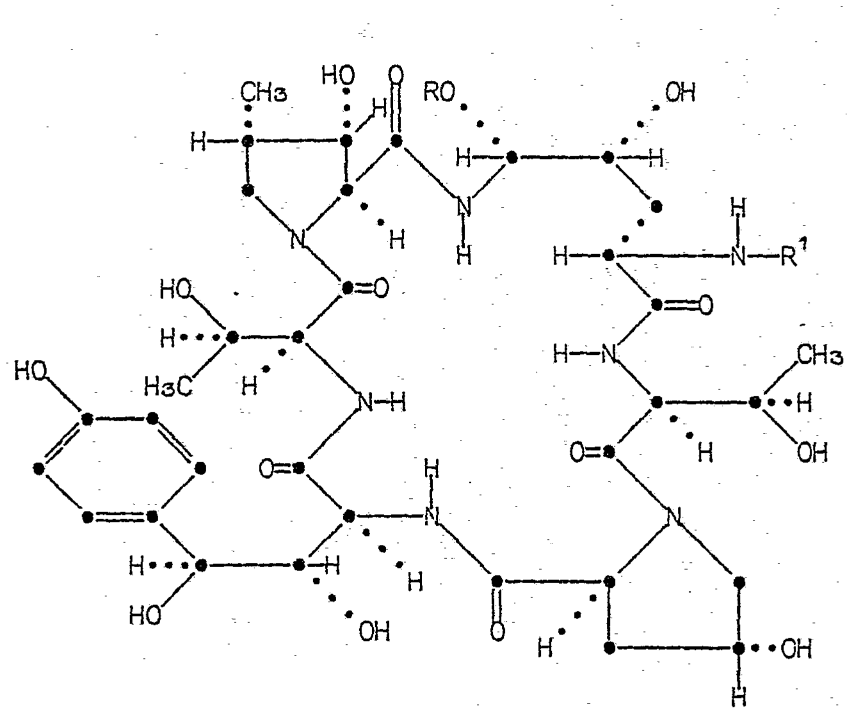

I

wherein $R^1$ is linoleoyl or stearoyl and R is $C_1-C_6$ alkyl, subject to the proviso that when R is methyl $R^1$ is linoleoyl, characterized by reacting a compound of Formula II

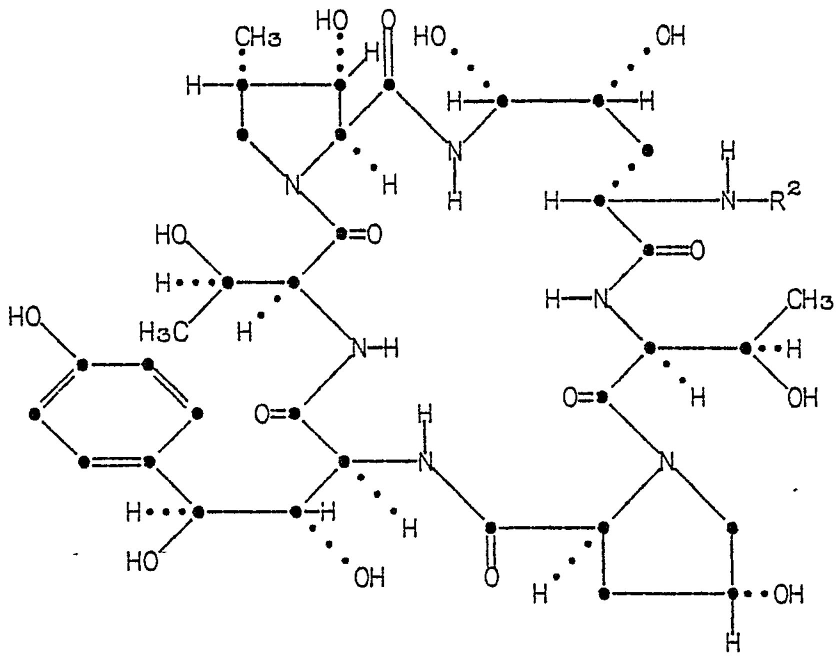

II

wherein $R^2$ is linoleoyl or stearoyl

A) when $R^2$ is linoleoyl, with

1) an alcohol of the formula ROH wherein R is defined as above; or

2) an alcohol of the formula $R^3OH$ wherein $R^3$ is $C_2-C_6$ alkyl;

B) when $R^2$ is stearoyl, with an alcohol of the formula $R^3OH$;

in a polar inert solvent or in an excess of the reactant alcohol at 0°C to 70°C. under acidic conditions; and when the alcohol $R^3OH$ is reacted with a compound of Formula II wherein $R^2$ is linoleoyl, optionally, reducing the linoleoyl group in the resulting compound to stearoyl.

8. The process of claim 7 for preparing the A-30912H-type antibiotic of formula I wherein $R^1$ is linoleoyl and R is $-CH_3$, that is antibiotic A-30912 factor H, characterized by reacting A-30912 factor A, dissolved in dimethylformamide with acidic methanol (HCl) at room temperature with overnight stirring followed by evaporation to dryness and purification by high pressure chromatography.

9. A pharmaceutical formulation which comprises the active ingredient, an A-30912H-type antibiotic which has the structural formula I as described in claim 1, associated with a pharmaceutically acceptable carrier therefor.

10. An A-30912H-type antibiotic which has the structural formula I as described in claim 1 for use as an antifungal agent for treating fungal infections in mammals.

11. An A-30912 H-type antibiotic according to any of claims 1 to 6 substantially free from impurity.

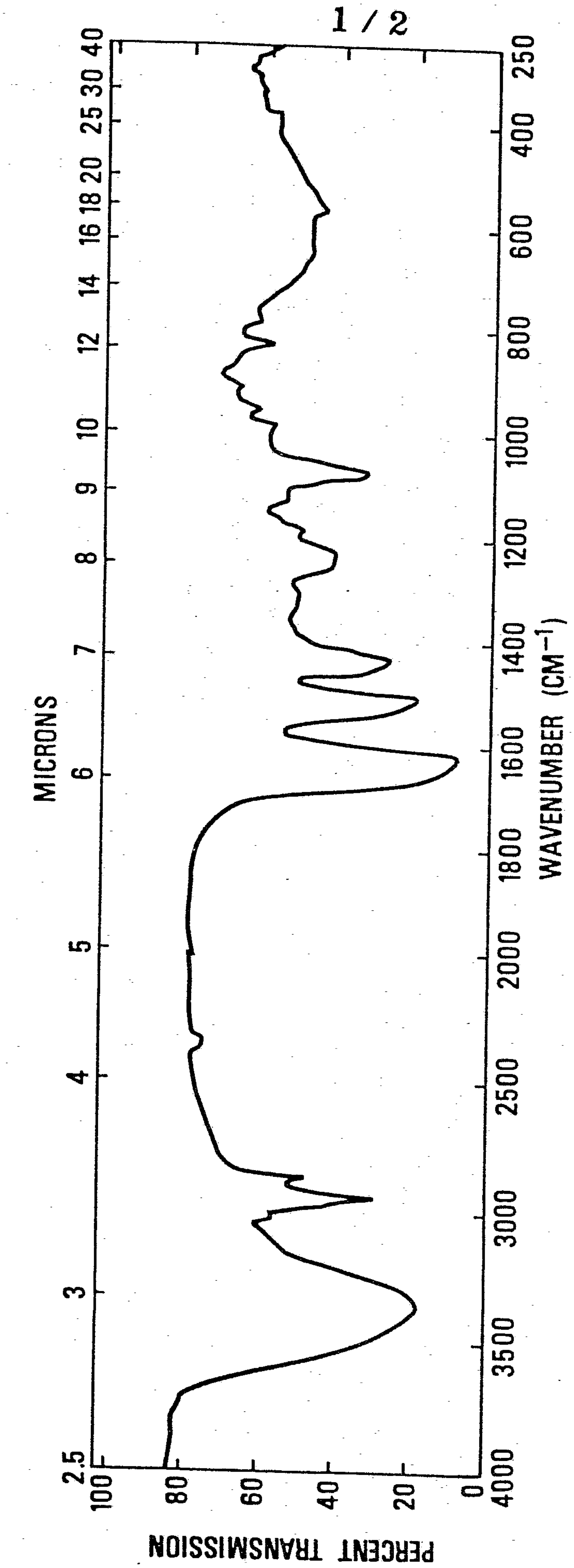
MICRONS
2.5
3
4
5
6
7
8
9
10
12
14
16 18 20 25 30 40
PERCENT TRANSMISSION
100
80
60
40
20
0
WAVENUMBER (CM$^{-1}$)
4000
3500
3000
2500
2000
1800
1600
1400
1200
1000
800
600
400
250

FIG. 1

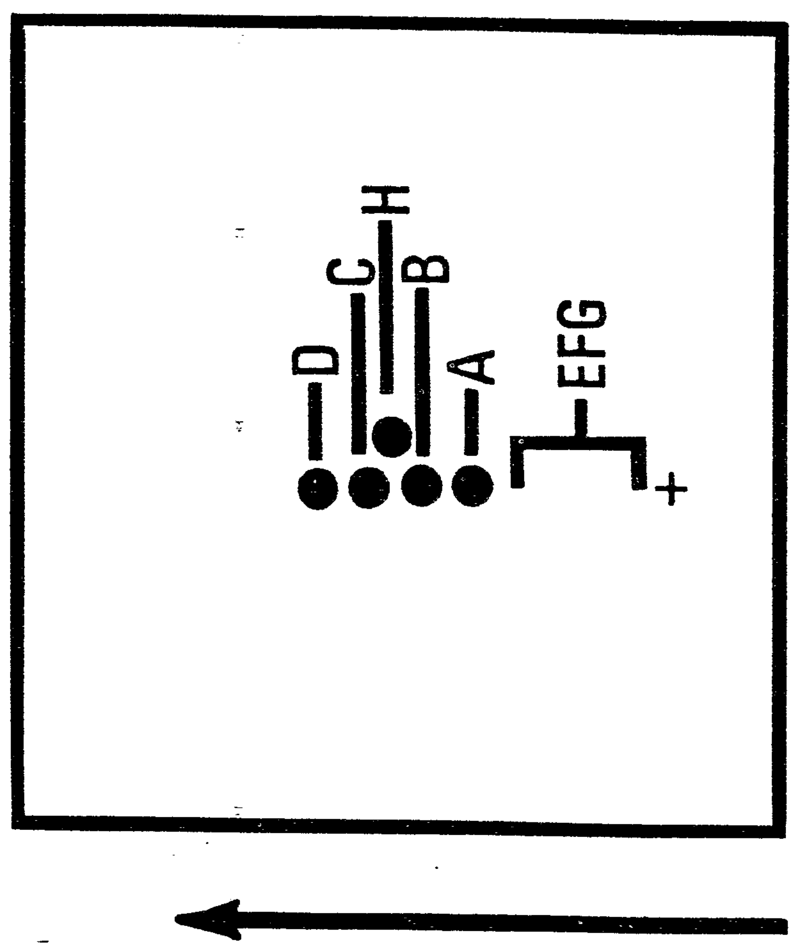


FIG. 2

0021685

European Patent Office

# EUROPEAN SEARCH REPORT

Application number
EP 80 30 1913.2

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | TETRAHEDRON LETTERS, Vol. 46, 1976<br>C. KELLER-JUSLEN et al. "Struktur des Cyclopeptid-Antibiotikums SL 7810 (= Echinocandin B)"<br>pages 4147 to 4150<br>* complete document *<br>-- | 1,9 | C 07 C 103/52<br>A 61 K 37/02 |
| O | HELVETICA CHIMICA ACTA , Vol 62, Fasciculus 4, 08. Juni 1979<br>R. TRABER et al. "Cyclopeptid-Antibiotika aus Aspergillus-Arten. Struktur der Echinocandine C und D"<br>pages 1252 to 1267<br>* page 1252, footnote 1;<br>page 1257, paragraph 1;<br>page 1264, paragraphs 3,4,5 *<br>---- | 1 | TECHNICAL FIELDS SEARCHED (Int.Cl.3)<br>A 61 K 37/02<br>C 07 C 103/52<br>C 07 D 487/14 |

CATEGORY OF CITED DOCUMENTS
X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| Berlin | 06-10-1980 | STOOS |

EPO Form 1503.1 06.78